# EUROPEAN PATENT APPLICATION

(11) **EP 4 741 418 A1**
(43) Date of publication of application: **13.05.2026**
(21) Application number: 24835152.0
(22) Date of filing: 21.05.2024
(51) Int. Cl.: C07K 14/47, C12N 15/12, C12N 15/70, C07K 1/34, A61K 38/17

(54) **KERATIN CF1, PREPARATION METHOD THEREFOR, PHARMACEUTICAL COMPOSITION COMPRISING SAME, AND USE THEREOF**

(30) Priority: 05.07.2023 CN 202310815809
(71) Applicant: Institute of Materia Medica, Chinese Academy of Medical Sciences, Beijing 100050 (CN)
(72) Inventor: YU, Shishan, Beijing 100050 (CN); WANG, Xiaoliang, Beijing 100050 (CN); MA, Shuanggang, Beijing 100050 (CN); WANG, Xiaojing, Beijing 100050 (CN); QU, Jing, Beijing 100050 (CN); WANG, Ling, Beijing 100050 (CN); LI, Mi, Beijing 100050 (CN); FENG, Nan, Beijing 100050 (CN); LIU, Yunbao, Beijing 100050 (CN); XU, Shaofeng, Beijing 100050 (CN); LI, Yong, Beijing 100050 (CN); CAI, Jie, Beijing 100050 (CN); WANG, Weiping, Beijing 100050 (CN); ZHANG, Mi, Beijing 100050 (CN)
(74) Representative: Santarelli
(86) International application number: PCT/CN2024/094334
(87) International publication number: WO 2025/007667

(57) **Abstract**

The present application relates to the technical field of medicines, and relates to keratin CF1, a preparation method therefor, a pharmaceutical composition comprising same, and a use thereof, and in particular to keratin CF1, a nucleic acid molecule encoding keratin CF1, an expression vector containing the nucleic acid molecule, a host cell containing the expression vector or a genome into which the nucleic acid molecule is integrated, a preparation method for keratin CF1, a pharmaceutical composition containing keratin CF1, and a use of keratin CF1, the nucleic acid molecule, the expression vector, the host cell or the pharmaceutical composition in preparation of medications for relieving fever, easing pain, relieving cough, eliminating phlegm, resisting convulsion, resisting epilepsy, reducing blood pressure, resisting inflammation, and resisting viruses.

## Description

### Technical field

The present invention pertains to the field of medical technology, relates to a keratin CF1, a nucleic acid molecule encoding the keratin CF1, an expression vector comprising the nucleic acid molecule, and a host cell comprising the expression vector or the nucleic acid molecule integrated in the genome, and preparation methods of the keratin CF1 and pharmaceutical compositions comprising the keratin and use of the keratin and the pharmaceutical composition in the manufacture of medicaments for antipyretic, analgesia, antitussive, expectorant, anticonvulsant, antiepileptic, hypotensive, anti-inflammatory, and antiviral.

### Background Art

Keratin is a kind of protein that is widely found in the epidermis of humans and animals, and it is the main component of hair, feathers, hoofs, shells, claws, horns, etc. It is an extremely important structural protein for connective tissue and plays a role in protecting the body.

Keratin exists widely in organisms and is a kind of renewable resource with great utilization value, but it has not been widely and effectively utilized. The main reasons are that keratin is not easy to dissolve in various solvents, and keratin is generally more resistant to protease hydrolysis than other proteins. Therefore, it is very difficult to extract and prepare natural keratin.

With the rapid development of modern biotechnology such as genomics, proteomics, genetic engineering, microbial engineering, etc., more and more genes are discovered. It is an important means to produce target proteins by utilizing the protein expression system for studying the biological function of genes or proteins.

It is novel and innovative and is not reported in other literatures to prepare the target keratin by utilizing the protein expression system, and further to study its structure and function.

### Summary of the invention

The technical problem solved by the present invention is to provide a keratin CF1, a nucleic acid molecule encoding keratin CF1, an expression vector comprising the nucleic acid molecule, and a host cell comprising the expression vector or the nucleic acid molecule integrated in the genome, and preparation methods of keratin CF1, pharmaceutical compositions comprising the keratin CF1, and use of the above-mentioned keratin CF1, nucleic acid molecule, expression vector, host cell, or pharmaceutical compositions in the manufacture of medicaments for antipyretic, analgesia, antitussive, expectorant, anticonvulsant, antiepileptic, hypotensive, anti-inflammatory, and antiviral.

In order to solve the technical problems of the present invention, the present invention provides the following technical solutions:
The first aspect of the technical solution of the present invention is to provide a keratin CF1, characterized in that the amino acid sequence of the keratin CF1 is as follows:
(1) the amino acid sequence shown in SEQ ID NO.1 in the sequence listing; or
(2) the amino acid sequence formed by substitution, deletion or addition of 1-35 amino acids in the amino acid sequence shown in SEQ ID NO.1 in the sequence listing that basically maintains the same biological function.

Further, conventional modification can be performed on keratin CF1; or a tag for detection or purification can be attached to keratin CF1.

Furthermore, the conventional modification includes acetylation, amidation, cyclization, glycosylation, phosphorylation, alkylation, biotinylation, fluorescent group modification, polyethylene glycol (PEG) modification, immobilization modification, sulfation, oxidation, methylation, deamination, formation of disulfide bonds or disulfide bonds breakage; The tag includes His6, GST, EGFP, MBP, Nus, HA, IgG, FLAG, c-Myc, Profinity eXact.

The second aspect of the technical solution of the present invention provides a nucleic acid molecule encoding the keratin CF1 of the first aspect.

Further, the nucleotide sequence of the nucleic acid molecule is:
(1) The nucleotide sequence shown in SEQ ID NO. 2 in the sequence listing.
(2) a nucleotide sequence obtained by sequence optimization based on the nucleotide sequence shown in SEQ ID NO.2.
(3) a nucleotide sequence complementary to the nucleotide sequence in (1) or (2) above-mentioned.

The third aspect of the technical solution of the present invention provides an expression vector, characterized in that the expression vector comprises the nucleic acid molecule described in the second aspect.

Further, the expression vector can be pET series, pUC series, pQE series, pBV series, pMAL series, pPIC9, pPIC9K, pHIL-S1, pPICZα/A, pYAM75P, pHIL-D2, pA0815, pPIC3K, pPICZ, pHWO10, pGAPZ, pGAPZa, pPIC3.5K, etc.; the preferred expression vector is the pET series vector; the most preferred expression vector is pET-28a(+).

The fourth aspect of the technical solution of the present invention provides a host cell, characterized in that the host cell comprises the expression vector of the third aspect or the nucleic acid molecule of the second aspect integrated in the genome.

Further, the host cell includes bacteria, yeast, aspergillus, plant cells, or insect cells.

Furthermore, the bacteria include *Escherichia coli* or yeast.

Competent host cells can be BL21 series, Transetta series, Rosetta series, DH5α series, JM series, Top series, Orgami series, Transl-T1, TG1, TB1; Y11430, MG1003, GS115 (AOX1), KM71, SMD1168, etc.; The preferred expression competent cells are BL21 (DE3), Transetta (DE3).

The fifth aspect of the technical solution of the present invention provides a method for preparing the keratin CF1 of the first aspect, which is characterized in that it comprises the following steps:
A. Synthesizing the nucleic acid molecule corresponding to the keratin CF1 described in the first aspect, ligating the nucleic acid molecule into a corresponding expression vector, transforming the expression vector into a host cell, culturing the host cell with the expression vector in fermentation equipment under certain conditions and inducing expression of keratin CF1 to obtain a crude protein solution containing keratin CF1.
B. Separating, purifying and drying the crude protein solution expressed in step A to obtain keratin CF1.

Further, in step A, the host cell is mainly selected from *Escherichia coli,* the keratin CF1 is expressed in inclusion bodies of *Escherichia coli,* and the fermentation equipment includes shake flask or fermentation tank.

Further, in step A, after inducing the expression of keratin CF1, using detergent to wash impurities, then using a urea solution to dissolve to obtain a crude protein solution.

Further, the medium in step A can be LB medium, TB medium, SB medium, SOB medium, SOC medium, PDA medium, YPD medium, rose bengal medium, salt Czapek Dox medium, DOBA medium, rice koji medium and its modified formula, etc.; for shake flask fermentation, LB medium and TB medium are preferred, with TB medium being the most preferred; for fermentation tank, LB medium and its modified formula are preferred.

Further, the inducer in step A can be IPTG, lactose, arabinose, etc.; preferably IPTG, lactose.

Further, in step A, the obtained fermentation broth is centrifuged to discard the supernatant; The precipitate is suspended in a buffer, followed by bacterial cell disruption and subsequent centrifugation to remove the supernatant; After the precipitate is washed with a detergent, it is then dissolved in a urea solution to obtain a CF1 crude protein solution.

Among them, the buffer is preferably buffer A, and its dosage is: fermentation broth volume: buffer A volume=1~100:1, preferably 10:1.

The detergents can be urea solution, guanidine hydrochloride solution, triton and buffer A, etc., preferably urea solution, most preferably 2M urea solution (containing 1% Triton) and 4M urea solution. The dosage is: fermentation broth volume: urea solution volume = 0.2~100:1, preferably 1~15:1.

The urea solution is preferably an 4M-8M urea solution, most preferably 8M urea solution, and its dosage is: fermentation broth volume: 8M urea volume=0.2~100: 1, preferably 2~15: 1.

Further, in step B, the method for separating and purifying includes ultrafiltration or microfiltration membrane technology purification method, column chromatography purification method, salting out method, and dialysis method.

Further, in step B, the method for separating and purifying is as follows:
(1) The dialysis method is to purify the crude protein solution obtained in step A by the dialysis method to obtain the target protein CF1 solution.
   The molecular weight cut-off of the dialysis bag can be 0.5-10kD, the preferred molecular weight cut-off of the dialysis bag is 3.5-10kD, the most preferred molecular weight cut-off of the dialysis bag is 10kD.
(2) The method for ultrafiltration and microfiltration is to purify the crude protein solution obtained in step A by membrane technology such as ultrafiltration membrane or microfiltration membrane to obtain a concentrated solution of the target protein CF1.
(3) The column chromatography method is to pass the crude protein solution obtained in step A through column chromatography, such as various exchange column or exclusion column chromatography, to separate and purify, to obtain the target protein CF1.

The preferred exclusion column is dextran gel column, Superdex 30 Increase, Superdex 75 Increase, Superdex 200 Increase, Superose 6 Increase, etc.; The preferred exchange column is an ion exchange resin column: anion exchange resin column, HiTrap Q FF, HiTrap Capto Q ImpRes, Capto Q ImpRes, HiTrap Capto Q, HiTrap DEAE, Toyopearl Q-650M Toyopearl SuperQ-650M, etc.; Cation exchange resin column: HiTrap SP FF, HiTrap Capto SP ImpRes, Capto SP ImpRes, HiTrap Capto SP, Toyopearl SP-650M, Toyopearl Super SP-650M.

As the eluent, commonly used eluents in the art can be used, such as water, salt solution. The salt solution includes sodium chloride solution, sodium dihydrogen phosphate solution, disodium hydrogen phosphate solution, sodium acetate, acetic acid, and the like.

(4) The salting-out method is to purify the crude protein solution obtained in step A by salting-out method to obtain the target protein CF1 suspension.

The salting-out agent can be ammonium sulfate, sodium sulfate, sodium chloride, magnesium chloride, aluminum sulfate, ammonium nitrate, ammonium chloride, magnesium sulfate, and the like. The preferred salting-out agent is ammonium sulfate and its aqueous solution. A saturated aqueous solution of ammonium sulfate is added to make the final concentration of ammonium sulfate reach 10-50%, preferably 20-30%, more preferably 25%.

The number of times of salting out is 1 to 3 times, preferably 2 times.

After salting out, the precipitate is washed by adding pure water for 2 to 5 times, preferably 3 times.

Further, the target protein CF1 solution purified in step B can be freeze-dried or vacuum-dried into dry powder, or the concentrated solution can be directly spray-dried into dry powder.

The sixth aspect of the technical solution of the present invention provides a pharmaceutical composition, characterized in that the pharmaceutical composition comprises the keratin CF1 described in the first aspect or the nucleic acid molecule described in the second aspect or the expression vector described in the third aspect or the host cell described in the fourth aspect and a pharmaceutically acceptable carrier or excipient.

The keratin obtained in the above-mentioned steps of the present invention can be freeze-dried or vacuum-dried into dry powder, or the concentrated liquid can be directly spray-dried into dry powder and then made into various dosage forms.

The present invention relates to a pharmaceutical composition, which comprises any keratin obtained in the above-mentioned steps and a pharmaceutically acceptable carrier.

The present invention also relates to a pharmaceutical composition comprising the keratin of the present invention as an active ingredient and a conventional pharmaceutical excipient or adjuvant. Generally, the keratin of the present invention accounts for 0.1-100.0% of the total weight of the pharmaceutical composition.

The present invention also provides a pharmaceutical composition, which comprises a pharmaceutical effective dose of protein as an active ingredient and a pharmaceutically acceptable carrier.

The pharmaceutical composition of the present invention can be prepared according to methods known in the art. When used for this purpose, if necessary, the protein of the present invention can be combined with one or more solid or liquid pharmaceutical excipients and/or adjuvants to prepare an appropriate administration form or dosage form that can be used as human drugs or Veterinary drugs.

The keratin of the present invention or the pharmaceutical composition comprising the same can be administered in a unit dosage form. The route of administration can be enteral or parenteral, such as oral, intramuscular, subcutaneous, nasal, oral mucosal, ocular, pulmonary, transdermal, vaginal, peritoneal and rectal, etc., oral administration is preferred.

The keratin protein of the present invention or the pharmaceutical composition comprising the same can be administered by injection. Injections include intravenous injection, intramuscular injection, subcutaneous injection, intradermal injection, intraperitoneal injection, and acupoint injection, etc.

The dosage form for administration can be a liquid dosage form, a solid dosage form or a semi-solid dosage form. Liquid dosage forms can be solutions (including true solutions and colloidal solutions), emulsions (including oil-in-water, water-in-oil and double emulsions), suspensions, injections (including water injections, powder injections and infusions), eye drops, nasal drops, lotion and liniment, etc. The solid dosage form can be tablets (including ordinary tablets, enteric-coated tablets, buccal tablets, dispersible tablets, chewable tablets, effervescent tablets, orally disintegrating tablets), capsules (including hard capsules, soft capsules, and enteric-coated capsules), granules, powders, pellets, dripping pills, suppositories, films, patches, air (powder) sprays, sprays, etc.; semi-solid dosage forms can be ointments, gels, pastes, etc.

The keratin of the present invention can be made into ordinary preparations, slow-release preparations, controlled-release preparations, targeted preparations, and various particle delivery systems.

In order to make a unit administration dosage form into a tablet, various excipients known in the art can be widely used, including diluents, binders, wetting agents, disintegrants, lubricants, glidants. The diluent can be starch, dextrin, sucrose, glucose, lactose, mannitol, sorbitol, xylitol, microcrystalline cellulose, calcium sulfate, calcium hydrogen phosphate, calcium carbonate, etc.; the wetting agents can be water, ethanol, isopropanol, etc.; the binder can be starch slurry, dextrin, syrup, honey, glucose solution, microcrystalline cellulose, acacia slurry, gelatin slurry, sodium carboxymethyl cellulose, methyl cellulose, hydroxypropyl methyl cellulose, ethyl cellulose, acrylic resin, carbomer, polyvinylpyrrolidone, polyethylene dipropyl alcohol, etc.; the disintegrant can be dry starch, microcrystalline cellulose, low-substituted hydroxypropyl cellulose, cross-linked polyvinylpyrrolidone, cross-linked sodium carboxymethyl cellulose, sodium carboxymethyl starch, sodium bicarbonate and citric acid, calcium carbonate, polyoxyethylene sorbitol fatty acid ester, dodecyl sodium sulfonate; the lubricant and glidant can be talc, silicon dioxide, stearate, tartaric acid, liquid paraffin, polyethylene glycol, etc.

The tablets can also be further made into coated tablets, such as sugar-coated tablets, film-coated tablets, enteric-coated tablets, or double-layer tablets and multilayer tablets.

In order to make the administration unit into a pill, various carriers known in the field can be widely used. Examples of carriers are, for example, diluents and absorbents, such as glucose, lactose, starch, cocoa butter, hydrogenated vegetable oil, polyvinylpyrrolidone, polyethylene glycol laurate, kaolin, talc, etc.; binders, such as Gum arabic, xanthan gum, gelatin, ethanol, honey, liquid sugar, rice paste or batter, etc.; disintegrants, such as agar powder, dried starch, alginate, sodium lauryl sulfonate, methyl cellulose, ethyl cellulose, etc..

In order to make the administration unit into a suppository, various carriers known in the field can be widely used. Examples of carriers are, for example, polyethylene glycol, lecithin, cocoa butter, higher alcohols, higher alcohol esters, gelatin, semisynthetic glycerides and the like.

In order to make the administration unit into a capsule, the keratin of the present invention as an active ingredient is mixed with the above-mentioned various carriers, and the resulting mixture is placed into hard gelatin capsules or soft capsules. The keratin of the present invention as an active ingredient can also be made into microcapsules, suspended in an aqueous medium to form a suspension, or filled into hard capsules or made into injections for application.

For example, the keratin of the present invention is prepared into injection preparations, such as solutions, suspension solutions, emulsions and freeze-dried powder injections. Such preparations can be aqueous or non-aqueous and may contain one and/or more pharmacodynamically acceptable carriers, diluents, binders, lubricants, preservatives, surfactants or dispersants. For example, the diluent can be selected from water, ethanol, polyethylene glycol, 1,3-propylene glycol, ethoxylated isostearyl alcohol, polyoxylated isostearyl alcohol, polyoxyethylene sorbitol fatty acid esters and the like. In addition, in order to prepare an isotonic injection, an appropriate amount of sodium chloride, glucose or glycerin can be added to the injection preparation. In addition, conventional auxiliary solvents, buffers, pH adjusters, etc. can also be added. These auxiliary materials are commonly used in this field.

In addition, if necessary, coloring agents, preservatives, flavors, corrigents, sweeteners or other materials can also be added to the pharmaceutical preparations.

In order to achieve the purpose of medication and enhance the therapeutic effect, the keratin or the pharmaceutical composition of the present invention can be administered by any known administration method.

The administration dosage of the keratin pharmaceutical composition of the present invention depends on many factors, such as the nature and severity of the disease to be prevented or treated, the gender, age, weight, personality and individual response of the patient or animal, the route of administration, the frequency of administrations and the purpose of treatment, therefore the therapeutic dose of the present invention can have a wide range of changes. Generally speaking, the dosage of the pharmaceutical ingredients of the present invention is well known to those skilled in the art. Appropriate adjustments can be made according to the actual amount of the drug contained in the final preparation of the keratin composition of the present invention to meet the requirement of the therapeutically effective amount and accomplish the preventive or therapeutic purpose of the present invention. The appropriate daily dosage range of the keratin of the present invention: the dosage of the keratin of the present invention is from 0.01 to 500 mg/kg body weight, preferably from 0.5 to 100 mg/kg body weight, more preferably from 1 to 50 mg/kg body weight, and most preferably from 2 to 30 mg/kg body weight. The above dosage can be administered in a single dosage form or divided into several, such as two, three or four dosage forms for administration, depending on the clinical experience of the administering doctor and the dosage regimens including the use of other treatments. The total dose required for each treatment can be divided into multiple or single doses for administration. The protein or pharmaceutical composition of the present invention can be taken alone or be used in combination with other therapeutic drugs or symptomatic drugs with adjusting the dose.

The seventh aspect of the technical solution of the present invention provides use of the keratin CF1 according to the first aspect or the nucleic acid molecule according to the second aspect or the expression vector according to the third aspect or the host cell according to the fourth aspect or the pharmaceutical composition according to the sixth aspect in the manufacture of medicaments for antipyretic, analgesia, antitussive, expectorant, anticonvulsant, antiepileptic, hypotensive, anti-inflammatory, and antiviral.

In order to accomplish the purpose of the present invention, the present invention takes the following technical solutions. Specifically, the preparation of keratin CF1 of the present invention includes the following steps:
(1) Synthesizing the nucleotide sequence and determining the accuracy of the sequence;
   The preferred nucleotide sequence is shown in SEQ ID No. 2.
(2) Transferring the nucleotide sequence into an expression vector;
   The expression vector can be pET series, pUC series, pQE series, pBV series, pMAL series, pPIC9, pPIC9K, pHIL-S1, pPICZα/A, pYAM75P, pHIL-D2, pA0815, pPIC3K, pPICZ, pHWO10, pGAPZ, pGAPZa, pPIC3.5K etc. The preferred expression vector is the pET series vector; the most preferred expression vector is pET-28a(+).
(3) Transfecting the expression vector into a host cell;
   The host cell can be E. coli or yeast; the preferred host cell is E. coli;
   Competent cells can be BL21 series, Transetta series, Rosetta series, DH5α series, JM series, Top series, Orgami series, Transl-T1, TG1, TB1; Y11430, MG1003, GS115 (AOX1), KM71, SMD1168, etc. The preferred competent cells for expression are BL21 (DE3), Transetta (DE3).
(4) Performing fermentation culture of the host cells to induce the expression of the target protein CF1 under appropriate conditions;
   Fermentation equipment can use shake flasks or fermentation tanks;
   The medium can be LB medium, TB medium, SB medium, SOB medium, SOC medium, PDA medium, YPD medium, rose bengal medium, salt Czapek Dox medium, DOBA medium, rice koji culture medium and their improved formulas, etc.; for shake flask fermentation, LB medium and TB medium are preferred, with TB medium being the most preferred; for fermentation tank, LB medium and its improved formulas are preferred.
   The inducer can be IPTG, lactose, arabinose, etc.; The preferred is IPTG, lactose.
(5) Enriching the Target protein CF1 product;
   Centrifuging the fermentation broth obtained in step (4), and discarding the supernatant; The precipitate is suspended in a buffer, followed by bacterial cell disruption and subsequent centrifugation to remove the supernatant; After the precipitation is washed with detergents, it is dissolved in a urea solution to obtain a CF1 crude protein solution.
   Among them, the buffer is preferably buffer A, and its dosage is: fermentation broth volume: buffer A volume=1~100: 1, preferably 5~10: 1;
   The detergent can be urea solution, guanidine hydrochloride solution, Triton, buffer A, etc., preferably urea solution, most preferably 2M urea solution (may contain 1% Triton). The dosage is: fermentation broth volume: urea solution volume =0.2~100: 1, preferably 1~15: 1; the wash times is 3~10 times, preferably 6 times.
   The urea solution is preferably an 4M-8M urea solution, most preferably 8M urea solution. Its dosage is: fermentation broth volume: 8M urea solution volume=0.2~100:1, preferably 2~15: 1.
(6) Separation and purification of target protein CF1.

The crude protein solution obtained in step (5) needs to be purified to obtain the target protein CF1. The purification can be carried out by dialysis, or ultrafiltration and microfiltration, or column chromatography, or salting out steps.
A. The dialysis step is to purify the crude protein solution obtained in step (5) by a dialysis method to obtain the target protein CF1 solution.
   The molecular weight cut-off of the dialysis bag can be 0.5-10kD, the preferred molecular weight cut-off of the dialysis bag is 3.5-10kD, and the most preferred molecular weight cut-off of the dialysis bag is 10kD.
B. The step of ultrafiltration and microfiltration is to purify the crude protein solution obtained in step (5) by membrane technology such as ultrafiltration membrane or microfiltration membrane to obtain the target protein CF1 concentrated solution.
C. The column chromatography step is to pass the crude protein solution obtained in step (5) through column chromatography, such as various exchange column or exclusion column chromatography, to separate and purify and to obtain the target protein CF1.

The preferred exclusion column is dextran gel column, Superdex 30 Increase, Superdex 75 Increase, Superdex 200 Increase, Superose 6 Increase, etc.; The preferred exchange column is ion exchange resin column: anion exchange resin column, HiTrap Q FF, HiTrap Capto Q ImpRes, Capto Q ImpRes, HiTrap Capto Q, HiTrap DEAE, Toyopearl Q-650M, Toyopearl SuperQ-650M, etc.; Cation exchange resin column, HiTrap SP FF, HiTrap Capto SP ImpRes, Capto SP ImpRes, HiTrap Capto SP, Toyopearl SP-650M, Toyopearl Super SP-650M. The most preferred is anion exchange resin column.

As the eluent, commonly used eluents in the art can be used, such as water, salt solution, the salt solution includes sodium chloride solution, sodium dihydrogen phosphate solution, disodium hydrogen phosphate solution, sodium acetate, acetic acid, and the like.

D. The salting-out step is to purify the crude protein solution obtained in step (5) by a salting-out method to obtain the target protein CF1 suspension.

The salting-out agent can be ammonium sulfate, sodium sulfate, sodium chloride, magnesium chloride, aluminum sulfate, ammonium nitrate, ammonium chloride, magnesium sulfate, and the like. The preferred salting-out agent is ammonium sulfate and its aqueous solution. A saturated aqueous solution of ammonium sulfate is added to make the final concentration of ammonium sulfate reach 10-50%, preferably 20-30%, more preferably 25%.

The number of times of salting out is 1 to 3 times, preferably 2 times.

After salting out, the precipitate is washed by adding pure water for 2 to 5 times, preferably 3 times.

The target protein CF1 solution purified from steps A to D can be freeze-dried or vacuum dried into dry powder, or the concentrated solution can be directly spray-dried into dry powder.

The beneficial technical effects of the present invention:
1. The protein of the present invention is a keratin obtained for the first time, and the preparation method of the present invention has the characteristics of high yield and high sample purity.
2. By studying the pharmacodynamics of the protein CF1 on the yeast-induced SD rat fever model in the invention, it proves that the protein CF1 has a significant effect in reducing the elevated body temperature of the model animals at 8 hours post-modeling; By studying the pharmacodynamics of the protein CF1 on the lipopolysaccharide (LPS) -induced SD rat fever model, it proves that the protein CF1 has a significant effect in reducing the body temperature of the model rats at 2 hours post-modeling.
3. By studying the pharmacodynamics of the protein CF1 on the ammonia water-induced mice cough in the invention, it proves that the protein CF1 can reduce the cough frequency and has antitussive effect.

### Brief Description of the Drawings

Figure 1: Effect of protein CF1 on the lipopolysaccharide (LPS) -induced rat fever model.
   (Compared with normal control group, ** P<0.01; Compared with the model group, # P<0.05, ## P<0.01)
Figure 2: Effect of protein CF1 on the yeast-induced rat fever model.
   (Compared with normal control group, ** P<0.01; Compared with the model group, # P<0.05, ## P<0.01)

### EXAMPLES

The following examples and pharmacological activity test examples are used to further illustrate the present invention, but this does not mean any limitation to the present invention.

The experimental methods in the following examples and pharmacological activity test examples are conventional methods unless otherwise specified; the experimental materials used, unless otherwise specified, are purchased from conventional biochemical reagent companies.

### Example 1 Preparation of protein CF1 crude solution A by Shake flask fermentation (TB medium)

The nucleotide sequence shown in SEQ ID No.2 was synthesized and transferred into the pET-28a(+) vector. It was confirmed by sequencing that an expression vector comprising the correct sequence was obtained. And the expression vector was transfected into BL21 (DE3) cells and expression competent host cells comprising the target nucleotide sequence were obtained. The expression competent host cells were added into LB medium and cultured in a shaker at 37°C and 220 rpm for 1 hour to obtain a recombinant strain.

The recombinant strain was dipped and streaked in LBA plates containing Kanamycin. The plates were placed upside down in a 37°C constant temperature incubator and incubated overnight for 16 hours.

400 ml of TB medium was prepared and divided into 2 bottles, 200 ml in each bottle. Kanamycin (final concentration 50µg/ml) was added to each bottle (200 ml) of TB medium. A single colony on the plate was taken and added to the TB medium and amplified and cultured overnight at 37°C and 220 rpm in the shaker to obtain seed liquid.

28.8 L of TB medium was prepared and divided into 144 bottles, 200 ml in each bottle. Each bottle (200 ml) of TB medium was added with Kanamycin (final concentration 50 µg/ml), then added with 2 ml of the seed liquid, and cultured in a shaker at 37°C and 220 rpm for 2-3 hours. The OD₆₀₀ was monitored, when the OD₆₀₀ reached about 1.0, an inducer was added to induce protein expression in a shaker, and the induction conditions were selected from the following table.

| | Inducer | Induction temperature | Induction time | Shaker speed |
|---|---|---|---|---|
| Inducing conditions | IPTG (Final concentration 0.5mM) | 16°C | 16 hours | 220 rpm |
| | | 25°C | 8 hours | |
| | | 37°C | 5 hours | |

All bottles of bacteria liquid were combined, centrifuged at 7000 rpm for 5 minutes, and the supernatant was discarded after sterilization. The precipitate was suspended in about 3 L of buffer, filtered with an 80-100 mesh screen, and the filtrate was crushed with a high-pressure crusher at a pressure of 800-1000 bar, twice, 2 minutes each time. The broken bacteria liquid was centrifuged at 7000 rpm for 30 minutes, the supernatant was discarded, and the precipitate (i.e. inclusion body) was obtained. The precipitate was washed with detergent (3 times with 1L of 2M urea-1% triton solution, twice with 1L of 3M urea solution), centrifuged and the supernatant was discarded. The precipitate was dissolved in 1L of 8M urea solution, and the crude protein solution A was obtained.

### Example 2 Preparation of protein CF1 crude solution B (other medium) in a fermentation tank

In Example 1, an expression vector was synthesized and sequenced to confirm that the expression vector comprising the sequence shown in SEQ ID No. 2 was obtained. The expression vector was transfected into BL21 (DE3) cells and expression-competent host cells comprising the target nucleotide sequence were obtained.

20 ml of LB medium was prepared, and 50µl of host cells comprising the target nucleotide sequence were added into 800µl of LB medium and were cultured in a shaker at 37°C and 220 rpm for 1 hour.

The above bacterial liquid was dipped and streaked into LBA plate containing Kanamycin, and then the plates were placed upside down and cultured in a constant temperature incubator at 37°C overnight for 16 hours.

10 ml of LB medium was obtained and added with Kanamycin (final concentration 50µg/ml). Single colonies were taken, added into LB medium and were cultured overnight at 37°C and 220 rpm for 15 hours, then the seed solution was obtained.

1L of medium as indicated in the table below was prepared and divided into 10 bottles, 100 ml each. Kanamycin (final concentration 50µg/ml) was added to each bottle (100 ml) of medium, and 1 ml of seed solution was added and cultured for 2-3 hours at 37 ° C and 220 rpm in a shaker. OD600 was monitored, and when OD600 reached about 1.0, the inducer IPTG (final concentration 0.5 mM) was added, and the protein expression was induced in a shaker at 37 ° C and 220 rpm.

| | |
|---|---|
| Culture medium | LB medium, SOB medium, SOC medium |

Each bottle of bacterial liquid was combined and was centrifuged at 10000 rpm for 10 minutes, and the supernatant was discarded after sterilization. The precipitate was suspended into about 100 mL of buffer, filtered with an 80-100 mesh screen, and the filtrate was crushed with a high-pressure crusher at a pressure of 800-1000 bar, twice, 2 minutes each time. The broken bacteria liquid was centrifuged at 10000 rpm for 30 minutes and the supernatant was discarded.

The precipitate was firstly added with 40 mL of detergent buffer A, washed and centrifuged, and the supernatant was discarded. Then, the precipitate was added with 40 mL of wash solution containing 2M urea solution (with 1% Triton), washed twice and centrifuged, and the supernatant was discarded. Subsequently, the precipitate was added with 40 mL of 3M urea solution, washed twice and centrifuged, and the supernatant was discarded after each wash. Finally, the precipitate was added with 40 mL of 8M urea solution, and the crude protein solution B was obtained.

### Example 3 Preparation of protein CF1 crude solution C in a fermentation tank

In Example 1, an expression vector was synthesized and sequenced to confirm that the expression vector comprising the sequence shown in SEQ ID No. 2 was obtained. The expression vector was transfected into BL21 (DE3) cells and expression-competent host cells comprising the target nucleotide sequence were obtained. The expression competent host cells were added into LB medium and cultured in a shaker at 37°C and 220 rpm for 1 hour to obtain a recombinant strain.

100µl of the recombinant strain was added to an LBA plate containing Kanamycin, spread evenly with a spreader until dry, and the plate was placed upside down in a constant temperature incubator at 37°C for overnight culture. Three single colonies were taken separately, streaked on plates containing Kanamycin, and then the plates were cultured overnight. After the verifications of three batches of shake flask fermentation and expression were confirmed to be correct, the strains were preserved with 15% glycerol and were divided into 0.8 ml for each to obtain a working cell bank, which was stored in a refrigerator at -80°C for later use.

100µl of 1 glycerol bacteria stock was taken out from the working cell bank, added with 40 ml of LB medium, added with Kanamycin (final concentration 50µg/ml), cultured in a shaker at 37°C and 220 rpm for 6 hours to obtain a first-level seed liquid.

1.2ml of the first-level seed liquid was taken, added to 120 ml of LB medium, added with Kanamycin (final concentration 50µg/ml), and then cultured in a shaker at 37°C and 220 rpm for 7 hours to obtain a second-level seed liquid.

3L of modified LB broth was added to a 5L fermentation tank, then added with 120 ml of the second-level seed liquid and 3 ml of Kanamycin (final concentration 50 µg/ml) and cultured at 37°C and 30% dissolved oxygen (series speed) for about 8 hours. The OD value was monitored to be about 20, and 3g lactose was used as an inducer. Induction was performed at 20°C, fed at a rate of 30ml/hour, and cultured at 20°C for 24 hours.

The bacteria liquid was centrifuged at 7000 rpm for 5 minutes and the supernatant was discarded after sterilization. The precipitate was suspended in about 600 mL of buffer A, filtered with an 80-100 mesh screen, and the filtrate was crushed with a high-pressure crusher at a pressure of 800-1000 bar, twice, 2 minutes each time. The broken bacteria liquid was centrifuged at 7000 rpm for 30 minutes and the supernatant was discarded.

The precipitate was added with 2M urea solution (containing 1% Triton) and washed three times, 1L each time; Then the precipitate was added with 1L of 3M urea solution, washed twice and centrifuged, and the supernatant was discarded. Subsequently, the precipitate was added with 1L of 8M urea solution, washed one time and centrifuged, and the supernatant was discarded. Finally, the precipitate was added with 2L of 8M urea solution, and the crude protein solution C was obtained.

### Example 4 Preparation of protein CF1 from protein crude solution A by dialysis

Protein crude solution A obtained in Example 1 was filtered with 0.45µm filter membrane, and the filtrate was combined. The filtrate was dialyzed with water, the molecular weight cutoff of the dialysis bag was 10kD. After 72 hours of dialysis, the inner liquid was freeze-dried to obtain the target protein CF1.

Confirmation of the protein CF1 structure-- Complete protein sequence analysis based on LC-MS/MS
Main materials: Acetonitrile, formic acid, ammonium bicarbonate, dithiothreitol (DTT), iodoacetamide (IAA), trypsin, chymotrypsin, Glu-C, Asp-N;
Main instruments: Capillary High Performance Liquid Chromatograph (Thermo Ultimate 3000), Electrospray-Combined Ion Trap Orbitrap Mass Spectrometer (Thermo Q Exative Hybrid Quadrupole-Orbitrap Mass Spectrometer).

### Methods and results:

Protein CF1 was subject to pre-treatments such as dissolution replacement, reductive alkylation, and various proteolysis to obtain enzyme-cleaved peptides. The solution of enzyme-cleaved peptides was analyzed by liquid chromatography tandem mass spectrometry. The original mass spectrometry file was retrieved from the protein database using Maxquant (1.6.2.10) for analysis data. The identification results determined that it was consistent with the target sequence of SEQ ID No.1.

### Example 5 Preparation of protein CF1 by purification from protein crude solution A through salting out method

The protein crude solution A was placed in a stirred container for salting out twice. Saturated ammonium sulfate solution was slowly added along the wall to make the final concentration of ammonium sulfate reach 25% or 50%. During the salting-out process, the protein was separated out. After the salting-out was completed, the solution was filtered, and the first salting out was finished. Then 400 ml of pure water was added into the precipitate to suspend, and a saturated solution of ammonium sulfate was slowly added along the wall again to make the final concentration of ammonium sulfate reach 25%. The second salting out and filtration were carried out, and the precipitate was the crude protein extract. The crude protein extract was washed three times with water: 200 ml of pure water was added to suspend, stirred, left standing, and filtered. After repeated three times, the precipitate was freeze-dried to obtain the target protein CF1.

The product protein CF1 was confirmed to have the same amino acid sequence as that of the protein prepared in Example 4 through the same structural confirmation method as in Example 4.

### Example 6 Preparation of protein CF1 by purification from protein crude solution B

The protein crude solution B obtained in Example 2 was purified by the following two methods:
The first method: dialysis;
The crude protein solution B was filtered with a 0.45µm membrane, the filtrate was dialyzed with water for more than 72 hours, and the inner solution was freeze-dried to obtain the target protein CF1.

| | |
|---|---|
| Dialysis bag | Molecular weight cutoff: 0.5kD, 3.5kD, 5kD, 10kD |

The second method: salting out;

The crude protein solution B was placed in a stirred container for salting out twice. Saturated ammonium sulfate solution was slowly added along the wall to make the final concentration of ammonium sulfate reach 25% or 50%. During the salting-out process, the protein was separated out. After the salting-out was completed, the solution was filtered, and the first salting out was finished. 400 ml of pure water was added into the precipitate to suspend, and then a saturated solution of ammonium sulfate was slowly added along the wall again to make the final concentration of ammonium sulfate reach 25%. The second salting out and filtration were carried out, and the precipitate was the crude protein extract. The crude protein extract was washed three times with water. It was suspended in 200 mL of pure water, stirred, allowed to stand, and filtered. After repeated three times, the precipitate was freeze-dried to obtain the target protein CF1.

The product protein CF1 obtained by the two methods was confirmed to have the same amino acid sequence as that the protein prepared in Example 4 through the same structural confirmation method as in Example 4.

### Example 7 Preparation of protein CF1 by purifying crude protein solution C.

Crude protein solution C was purified by microfiltration membrane technology: 20 nm or 50 nm ceramic membrane core was used for repeated microfiltration to remove urea; The inner liquid was freeze-dried to obtain the target protein CF1.

The product protein CF1 was confirmed to have the same amino acid sequence as the protein prepared in Example 4 through the same structural confirmation method as in Example 4.

### Pharmacological test

Experimental example 1 The pharmacodynamic test of protein CF1 (the protein in Example 7) on lipopolysaccharide (LPS) -induced SD rat fever model.
Animals: Male SD rats, weight: 230-260 grams;
Drugs: lipopolysaccharide (LPS, SIGMA L-2880), aspirin (SIGMA A2093), protein CF1;
Instruments: electronic balance (SARTORIUS BP121S), electronic clinical thermometer (CITIZEN CT-513W).

Experiment grouping:
Normal control group;
Model group: lipopolysaccharide fever model group;
Positive control group: Aspirin 300 mg/kg group;
Protein CF1, 10mg/kg group, 50mg/kg group.
Method: the method for replicating the rat fever model via intraperitoneal injection of lipopolysaccharide.

Preparation of experimental animals: After the experimental animals were adapted to the experimental environment (temperature 22°C±2°C, relative humidity 50%±2%) for 1 day, the rectal temperature was measured for pre-adaptation at 8:00 in the morning and 15:00 respectively. The animals were fasted for 12 hours before the experiment with water provided ad libitum, and their bowels were emptied before the rectal temperature was measured. Before each temperature measurement, the probe of the electronic thermometer was applied with Vaseline and inserted 2 cm into the rat's rectum (a marker was made at 2 cm to ensure consistent insertion depth each time). The body temperature was recorded after readings stabilized.

Intraperitoneal Injection of Lipopolysaccharide to replicate the rat fever model: Before model establishment, the body temperature of rats was measured. Qualified rats with body temperatures of 36.2-37.3 °C were selected and randomly grouped, with 8 rats per group. After oral administration of aspirin and different doses of protein CF1, lipopolysaccharide (20µg/kg, 2 ml/kg) was immediately intraperitoneally injected. The normal control group was intraperitoneally injected with the same volume of normal saline. The body temperatures of rats were monitored 2 hours later and for a total of 8 hours.

### Data statistics:

Based on the body temperature values measured at each time point on the day of the experiment, the mean, standard deviation and standard error of body temperature of rats in each group were calculated. The TTEST test was applied to compare the data of each group between groups., and P<0.05 was considered as a significant difference.

### Experiment results:

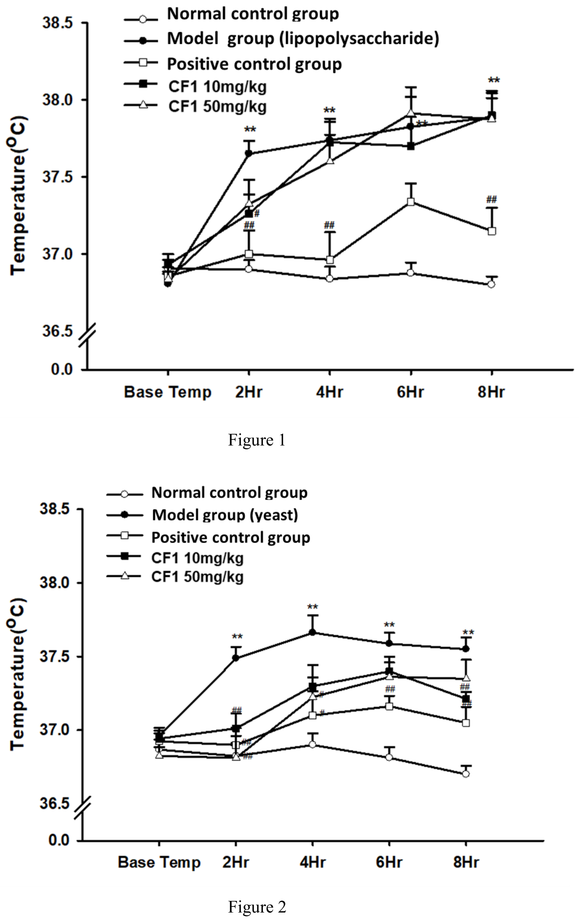

After oral administration of aspirin (300mg/kg) and protein CF1 (10mg/kg, 50mg/kg), 20µg/kg lipopolysaccharide was immediately intraperitoneally injected for modeling. The body temperatures of the animals were monitored at 2 hours, 4 hours, 6 hours, and 8 hours after modeling, respectively. The results are shown in Table 1 and Figure 1.

**Table 1. Effects of test drugs on lipopolysaccharide (LPS) induced fever in rats**

| Group | N | Basal body temperature (°C) | Body temperature 2 hours after modeling (°C) | Body temperature 4 hours after modeling (°C) | Body temperature 6 hours after modeling (°C) | Body temperature 8 hours after modeling (°C) |
|---|---|---|---|---|---|---|
| Normal control group | 8 | 36.9±0.1 | 36.9±0.1 | 36.8±0.1 | 3 6.9±0.1 | 36.8±0.1 |
| Model group | 8 | 36.8±0.1 | 37.7±0.1** | 37.7±0.1** | 37.8±0.2** | 37.9±0.2** |
| Positive control group | 8 | 36.9±0.1 | 37.0±0.2## | 37.0±0.2## | 37.3±0.1 | 37.2±0.1## |
| CF1 10mg/kg | 8 | 36.9±0.1 | 37.3±0.1# | 37.7±0.2 | 37.7±0.2 | 37.9±0.1 |
| CF1 50mg/kg | 8 | 36.8±0.05 | 37.3±0.2 | 37.6±0.2 | 37.9±0.2 | 37.9±0.1 |

| | | | | | | |
|---|---|---|---|---|---|---|
| (Compared with the normal control group; ** P<0.01; compared with the model group, # P<0.05, ## P<0.01) | | | | | | |

### Experimental conclusions:

After oral administration of aspirin (300mg/kg) and protein CF1 (10mg/kg, 50mg/kg), 20µg/kg lipopolysaccharide was immediately intraperitoneally injected for modeling. The body temperatures of the animals were monitored at 2 hours, 4 hours, 6 hours, 8 hours after modeling, respectively. The results showed that:
1) Intraperitoneal injection of 20µg/kg lipopolysaccharide can successfully induce elevated body temperature in rats. The model group shows significantly increased body temperature at 2 hours, 4 hours, 6 hours, and 8 hours after modeling. Compared with the normal group, P < 0.05 indicates a statistical difference and the model is stable.
2) The positive control group drug aspirin can effectively inhibit the increase of body temperature in model rats at 2 hours, 4 hours, 6 hours, and 8 hours after modeling. Compared with the model group, P < 0.05 indicates a statistical difference and the performance of the positive control drug is relatively stable.
3) The 10mg/kg dose group of protein CF1 can significantly reduce the body temperature of model rats at 2 hours after modeling. Compared with the model group, *P <* 0.05 indicates a statistical difference.

Experimental example 2 The pharmacodynamic test of protein CF1 (the protein in Example 7) on the yeast-induced SD rat fever model
Animals: male SD rats, weight 230-260 grams;
Medicines: yeast (OXOID LP0021), Aspirin (SIGMAA2093), protein CF1;
Instruments: electronic balance (SARTORIUS BP121S), electronic clinical thermometer (CITIZEN CT-513W).

Experiment grouping:
Normal control group;
Model group: yeast-induced fever model;
Positive control group: Aspirin 300 mg/kg group;
Protein CF1, 10 mg/kg group, 50 mg/kg group.

### Method:

Preparation of experimental animals: After the experimental animals were adapted to the experimental environment (temperature 22°C±2°C, relative humidity 50%±2%) for 1 day, the rectal temperature was measured for pre-adaptation at 8:00 in the morning and 15:00 respectively. The animals were fasted for 12 hours before the experiment with water provided ad libitum, and their bowels were emptied before the rectal temperature was measured. Before each temperature measurement, the probe of the electronic thermometer was applied with Vaseline and inserted 2cm into the rat's rectum (a marker was made at 2 cm to ensure consistent insertion depth each time). The body temperature was recorded after readings stabilized.

Subcutaneous injection of dry yeast to replicate the rat fever model: Before model establishment, the body temperature of rats was measured. Qualified rats with body temperatures of 36.2-37.3 °C were selected and randomly grouped, with 8 rats per group. After oral administration of aspirin and different doses of protein CF1, 20% yeast suspension (10ml/kg) was immediately subcutaneously injected. The normal control group was subcutaneously injected with the same volume of normal saline. The body temperatures of rats were monitored 2 hours later, once every 2 hours, for a total of 8 hours.

### Data Statistics:

Based on the body temperature values measured at each time point on the day of the experiment, the mean, standard deviation and standard error of body temperature of rats in each group were calculated. The TTEST test was applied to compare the data of each group between groups., and P<0.05 was considered as a significant difference.

### Experimental results:

After oral administration of aspirin (300mg/kg) and protein CF1 (10mg/kg, 50mg/kg), 20% yeast suspension was immediately subcutaneously injected for modeling. The body temperatures of the animals were monitored at 2 hours, 4 hours, 6 hours, and 8 hours after modeling, respectively. The results are shown in Table 2 and Figure 2.

**Table 2. Effects of the tested drugs on the yeast-induced fever model in rats**

| Group | N | Basal body temperature (°C) | Body temperature 2 hours after modeling (°C) | Body temperature 4 hours after modeling (°C) | Body temperature 6 hours after modeling (°C) | Body temperature 8 hours after modeling (°C) |
|---|---|---|---|---|---|---|
| Normal control group | 8 | 36.9±0.1 | 36.8±0.1 | 3 6.9±0.1 | 36.8±0.1 | 36.7±0.1 |
| Model group | 8 | 37.0±0.1 | 37.5±0.1** | 37.7±0.1** | 37.6±0.1** | 37.6±0.1** |
| Positive control group | 8 | 36.9±0.1 | 36.9±0.1## | 37.1±0.2# | 37.2±0.1## | 37.1±0.1## |
| CF1 10mg/kg | 8 | 36.9±0.1 | 37.0±0.1## | 37.3±0.1 | 37.4±0.1 | 37.2±0.05## |
| CF1 50mg/kg | 8 | 36.8±0.1 | 36.8±0.1## | 37.2±0.1# | 37.4±0.1 | 37.4±0.1 |

| | | | | | | |
|---|---|---|---|---|---|---|
| (Compared with the normal control group, ** P<0.01; compared with the model group, # P<0.05, ## P<0.01) | | | | | | |

### Experimental conclusions:

After oral administration of aspirin (300mg/kg) and protein CF1 (10mg/kg, 50mg/kg), 20% yeast suspension was immediately subcutaneously injected for modeling. The body temperatures of the animals were monitored at 2 hours, 4 hours, 6 hours, 8 hours after modeling, respectively. The results showed that:
1) The rats in model group exhibit significantly elevated body temperatures at 2 hours, 4 hours, 6 hours, and 8 hours post-modeling. Compared with the normal group, *P* < 0.05 indicates a statistical difference and the model is successfully established and is stable and reliable.
2) The positive control group drug aspirin can effectively inhibit the increase of body temperature in model rats at 2 hours, 4 hours, 6 hours, and 8 hours after modeling. Compared with the model group, P < 0.05 indicates a statistical difference and the performance of the positive control drug is relatively stable.
3) The 10mg/kg dose group of protein CF1 can significantly inhibit the temperature elevation in model rats at 2 hours and 8 hours post-modeling. Compared with the model group, *P* < 0.05 indicates a statistical difference.

Experimental example 3 The pharmacodynamic test of protein CF1 (the protein in Example 7) of antitussive effect on ammonia water-induced cough in mice
Animals: male ICR mice;
Drug and reagents: dextromethorphan hydrobromide, ammonia, 0.2% CMC-Na, protein CF1;
Instruments: Compressed nebulizer (403T), balance (XS105DU).

Experiment grouping:
Solvent control group;
Dextromethorphan 15 mg/kg group;
Protein CF1, 20 mg/kg group, 50 mg/kg group.

### Methods:

Model establishment and drug administration: Mice were orally administered dextromethorphan or varying doses of protein CF1 (administration volume: 10 ml/kg). The solvent control group were administered an equal volume of distilled water. After 1 hour, mice were placed in a sealed chamber and atomized with 10% ammonia water for 10 seconds. The cough latency period and cough frequency within 2 minutes were then observed and recorded.

Data processing: The oral administration time point, the nebulization exposure time point, the cough latency period, and the cough frequency within 2 minutes were recorded respectively. The cough latency period refers to the time interval of seconds required from the start of ammonia nebulization to the occurrence of coughing. The manifestation of coughing in mice is characterized by the contraction of their abdominal muscles (chest retraction) and the opening of their mouths wide. The mean and standard error of data in each group were calculated. The TTEST test was applied to compare the model group with the other groups. P <0.05 was considered as a significant difference.

### Experimental results:

Dextromethorphan (15 mg/kg) and different doses of protein CF1 (20 mg/kg, 50 mg/kg) was administered in advance. One hour later, mice were placed in a sealed chamber and were atomized with 10% ammonia water for 10 seconds. Then, the cough latency period and the cough frequency within 2 minutes of the mice were observed and recorded. The results are shown in Table 3.

**Table 3. Antitussive effect experiment of tested drugs on ammonia water-induced cough in mice (X±SEM)**

| Group | N | Latency period (s) | P | Cough frequency | P |
|---|---|---|---|---|---|
| Solvent control group | 9 | 30.0±1.8 | | 65.5±3.8 | |
| Dextromethorphan 15 mg/kg | 9 | 45.4±2.1** | 0.001 | 36.8±2.8** | 0.001 |
| CF1 20mg/kg | 9 | 34.6±3.1 | 0.214 | 50.1±2.8** | 0.006 |
| CF1 50mg/kg | 9 | 35.1±4.6 | 0.289 | 56.4±4.2 | 0.135 |

| | | | | | |
|---|---|---|---|---|---|
| (Compared with the solvent control group, * P<0.05, ** P<0.01) | | | | | |

### Experimental conclusion:

1) The experimental results demonstrate that compared to the solvent control group, the dextromethorphan group exhibits significant improvements in terms of latency period and cough frequency, with P<0.05, which is statistically significant.
2) Compared with the solvent control group, the CF1 (20 mg/kg) dose group shows a statistically significant difference in term of cough frequency, but the effect is weak.

## Claims

1. A keratin CF1, **characterized in that** the amino acid sequence of the keratin CF1 is:
(1) the amino acid sequence set forth in SEQ ID NO.1 in the sequence listing; or
(2) an amino acid sequence formed by substitution, deletion or addition of 1-45 amino acids in the amino acid sequence set forth in SEQ ID NO.1 in the sequence listing that basically maintains the same biological function.

2. The keratin CF1 according to claim 1, **characterized in that** the keratin CF1 can comprise a conventional modification; or be linked with a tag for detection or purification; or be homologous to keratin CF1.

3. The keratin CF1 according to claim 2, **characterized in that** the conventional modification comprises acetylation, amidation, cyclization, glycosylation, phosphorylation, alkylation, biotinylation, fluorescent group modification, polyethylene glycol (PEG) modification, immobilization modification, sulfation, oxidation, methylation, deamination, formation of disulfide bond(s) or breakage of disulfide bond(s); the tag comprises His6, GST, EGFP, MBP, Nus, HA, IgG, FLAG, c-Myc, Profinity eXact.

4. A nucleic acid molecule encoding the keratin CF1 of any one of claims 1 to 3.

5. The nucleic acid molecule according to claim 4, **characterized in that** the nucleotide sequence of the nucleic acid molecule is:
(1) the nucleotide sequence set forth in SEQ ID NO. 2 in the sequence listing;
(2) a nucleotide sequence obtained by sequence optimization based on the nucleotide sequence set forth in SEQ ID NO.2; or
(3) a nucleotide sequence complementary to the nucleotide sequence in (1) or (2) above-mentioned.

6. An expression vector, **characterized in that** the expression vector comprises the nucleic acid molecule of any one of claims 4-5.

7. A host cell, **characterized in that** the host cell comprises the expression vector of claim 6 or the nucleic acid molecule of any one of claims 4-5 integrated in the genome.

8. The host cell according to claim 7, **characterized in that** the host cell comprises bacteria, yeast, Aspergillus, plant cell, or insect cell.

9. The host cell according to claim 8, **characterized in that** the bacteria comprises *Escherichia coli.*

10. A method for preparing the keratin CF1 according to any one of claims 1 to 3, **characterized in that** it comprises the following steps:
A. synthesizing a nucleic acid molecule corresponding to the keratin CF1 according to any one of claims 1 to 3, ligating the nucleic acid molecule to a corresponding expression vector, transforming the expression vector into a host cell, culturing the host cell with the expression vector in a fermentation equipment under certain conditions and inducing the expression of keratin CF1 to obtain a crude protein solution comprising keratin CF1;
B. separating, purifying and drying the crude protein solution expressed in step A to obtain keratin CF1.

11. The method according to claim 10, **characterized in that**, in step A, the host cell is mainly selected from *Escherichia coli,* the keratin CF1 is expressed in inclusion bodies of *Escherichia coli,* and the fermentation equipment includes shake flask or fermentation tank.

12. The method according to claim 10, **characterized in that**, in step A, after inducing the expression of keratin CF1, removing impurities by using a detergent, dissolving keratin CF1 in a solution to obtain a crude protein solution.

13. The method according to claim 10, **characterized in that**, in step B, the method of separating and purifying comprises ultrafiltration or microfiltration membrane technology purification method, column chromatography purification method, salting out method, and dialysis method.

14. A pharmaceutical composition, **characterized in that** the pharmaceutical composition comprises the keratin CF1 according to any one of claims 1 to 3 and a pharmaceutically acceptable carrier or excipient.

15. Use of the keratin CF1 according to any one of claims 1 to 3 or the nucleic acid molecule according to any one of claims 4-5 or the expression vector according to claims 6 or the host cell according to any one of claims 7-9 or the pharmaceutical composition according to claim 14 in the manufacture of a medicament for antipyretic, analgesia, antitussive, expectorant, anticonvulsant, antiepileptic, hypotensive, anti-inflammatory, and antiviral.
